# EUROPEAN PATENT APPLICATION

(11) **EP 3 146 974 A1**
(43) Date of publication of application: **29.03.2017**
(21) Application number: 15306499.3
(22) Date of filing: 25.09.2015
(51) Int. Cl.: A61K 35/74, G01N 33/569

(54) **BIOMARKERS FOR EARLY DETERMINATION OF SEVERITY OF INFLUENZA RELATED DISEASE**

(71) Applicant: UNIVERSITE CLAUDE BERNARD - LYON 1, 69622 Villeurbanne Cédex (FR); Hospices Civils de Lyon, 69229 Lyon Cedex 02 (FR); University of Washington, Seattle, WA 98195 (US)
(72) Inventor: JOSSET, Laurence, 69003 Lyon (FR); PICHON, Maxime, 69007 Lyon (FR); LINA, Bruno, 69006 Lyon (FR); KATZE, Michael, Seattle, WA (US); LANGEVIN, Stanley, Seattle, WA (US)
(74) Representative: Nony

(57) **Abstract**

The present invention relates to the use of at least one biomarker for predicting the severity of a disease caused by the infection of an individual with an influenza virus, wherein said biomarker is selected in a group comprising (i) the alpha diversity value of the microbiome present in a respiratory sample of said individual and (ii) the microbiome profile of the said respiratory sample.

## Description

The present invention relates to the field of influenza infections, to the disease caused by said viral infections, and to biomarkers for predicting the severity of said disease in individuals infected by influenza virus.

### PRIOR ART

Influenza, commonly known as "the flu", designates a contagious disease affecting humans and other animals that is caused by an infection with at least one of the influenza viruses, chosen among type A, B and C influenza viruses and related mutants. Influenza A viruses may infect many animal species in addition to humans, while the influenza B and C viruses infect mainly humans.

Influenza infection rapidly spreads during seasonal epidemics and imposes considerable burden of hospitalization. The World Health Organization estimates that the annual attack rate of seasonal influenza is between 5%-10% in adults and 20%-30% in children, causing between 3 to 5 million cases of severe illness, and between 250 000 to 500 000 deaths globally annually.

Influenza viruses attack the respiratory tract in humans: the nose, throat, and lungs. The most common symptoms of influenza disease include: a high fever, runny nose, sore throat, muscle pains, headache, coughing, and intense tiredness. These symptoms typically begin two days after exposure and infection by the virus, and most last about a week.

Symptoms of the influenza-caused disease can be mild to severe. Even if most people recover from influenza disease within a week, some will develop complications which can be life-threatening and result in death.

Therefore, when the infection with an influenza virus is confirmed in an individual, the clinical decision to take is whether the individual should be admitted to hospital for a "severe disease", or return home if the disease is "mild".

From the clinical perspective, failure to identify patients with an influenza infection that have a higher risk of developing a severe disease delays the delivery of treatment appropriate for such high risk patients, and may have profound consequences for the recovery and long-term health of the patient. It has been reported that a delay of one day from onset of symptom to hospital admission increased the risk of death from influenza-caused disease by 5.5%.

Consequently, clinicians need a test that can assist them to evaluate the clinical risk associated with an influenza infection in an individual.

Several risk factors for severe disease, following seasonal influenza infection, have been identified such as: age superior to 65 years or inferior to 2 years, some pre-existing medical condition (such as chronic heart, lung, kidney, liver, blood or metabolic diseases or immunosuppression), pregnancy and obesity.

Nevertheless, the influenza-caused disease can be severe even in individuals without any of the already identified underlying risks.

To assess the severity of influenza infection, previous methods involved testing the viral load in a respiratory sample of an infected individual, as the extent of viral replication has been thought to correlate with disease severity. However, further studies on respiratory samples have shown that viral loads are the same regardless of disease severity (To *et al.,* 2010).

Influence of genetic factors has been investigated: for example, the H1N1 influenza strain causes severe pulmonary illness in a small number of exposed individuals; it has been shown that four risk single-nucleotide polymorphisms were significantly associated with this severe pulmonary illness (Zuniga *et al.,* 2012).

Similarly, the severity of influenza A H1N1 virus infection is associated with a single-nucleotide polymorphism "rs12252-C allele", which alters the function of interferon-induced transmembrane protein-3. This allele is rare in Caucasians but common in Han Chinese population (Zhang *et al.,* 2012).

The patent application WO 2014/008545 discloses a method for the identification of patients suspected to have an influenza infection, based on the measure of the interferon alpha inducible protein 27 (IFI27) in a biological sample. Moreover, this document suggests that a screening in patients who are admitted to hospital due to an influenza infection, to predict if the patient state might further deteriorate, could be performed when an elevated level of expression of the interferon alpha inducible protein 27 (IFI27) is observed in a biological sample from said patient.

Besides the teaching of this patent application, up to now, no other biomarker for predicting the severity of a disease caused by the infection of an individual with an influenza virus has been identified, according to the inventors' knowledge.

However, early prediction of influenza severity is critical for directing appropriate medical treatment and enhancing patient survival.

Therefore, there is still a need for biomarkers useful for identifying individuals at highest risk of severe outcomes, as early as possible, for protecting them with adapted therapeutic measures.

### SUMMARY OF THE INVENTION

The invention concerns the use of at least one biomarker for predicting the severity of a disease caused by the infection of an individual with an influenza virus, wherein said biomarker is selected in a group comprising:
i. the alpha diversity value of the microbiome present in a respiratory sample of said individual, and
ii. the microbiome profile of the said respiratory sample.

The present invention also concerns a method for predicting the severity of a disease caused by the infection of an individual with an influenza virus, comprising the steps of:
a) determining the alpha diversity value of the microbiome present in a respiratory sample from the tested individual, and
b) comparing the alpha diversity value determined at step a) to a reference alpha diversity value.

The present invention also concerns a method for predicting the severity of a disease caused by the infection of an individual with an influenza virus, comprising the steps of:
a) determining the microbiome profile present in a respiratory sample from the tested individual, and
b) comparing said microbiome profile determined in step a) to a reference respiratory microbiome profile.

With the methods according to the invention, the individuals infected with an influenza virus are classified into two classes of individuals:
- influenza-infected individuals with no clinical risk, developing a mild influenza disease; said individuals should be treated at home with no specific measures;
- influenza-infected individuals at risk of developing a severe disease, in particular a respiratory and/or a neurological complication; said individuals should be hospitalized as soon as the clinical risk is established, for an adequate treatment.

This categorization of individuals, preferentially at an early stage of the infection, especially when the individuals have been presenting influenza disease symptoms for about 24 to 72 hours, allow the clinicians to adapt their therapeutic strategy to the clinical risk evaluated for each infected individual.

The present invention also relates to the use of a bacterium or an extract thereof, as a probiotic, in the treatment and/or prevention of a severe disease in an individual infected with an influenza virus, wherein said bacterium has been identified as being present in a lower relative abundance in a microbiome profile present in a respiratory sample from an individual infected with an influenza virus, and presenting a severe disease, compared to a reference microbiome profile present in at least one respiratory sample of an individual infected with an influenza virus, and presenting a mild disease.

The present invention also relates to the use of an antibiotic specific of a bacterium, in the treatment and/or prevention of a severe disease in an individual infected with an influenza virus, wherein said bacterium has been identified as being present in a higher relative abundance in a microbiome profile present in a respiratory sample from an individual infected with an influenza virus, and presenting a severe disease, compared to a reference microbiome profile present in at least one respiratory sample of an individual infected with an influenza virus, and presenting a mild disease.

### BRIEF DESCRIPTION OF THE DRAWINGS

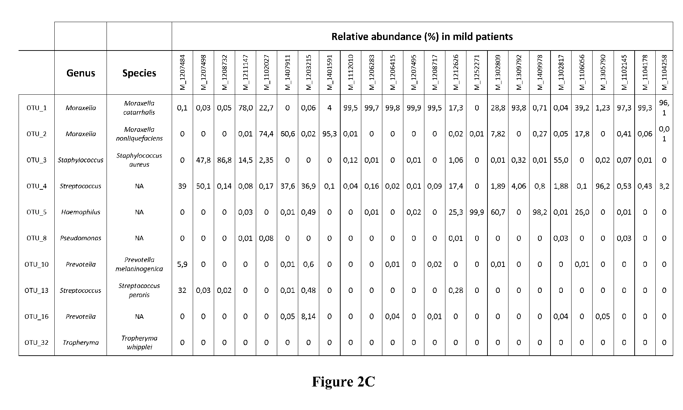

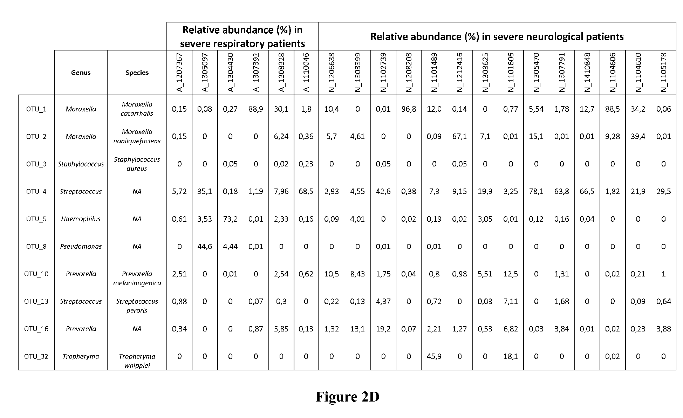

**Figure 1** **:** Total number of reads per OTUs and per samples. The Y axis represents a log10 scale: 1e+01 = 10, 1e+03=1000, 1e+05 = 100000
   A. Reads distribution for the 308 identified operational taxonomic units (OTUs).
   B. Reads distribution per sample. The black dashed line shows the cut-off of 50,000 high-quality *16S DNA* sequence reads per sample. All samples passed the cut-off.
**Figure 2** **:** Respiratory microbiome composition associated with each respiratory sample of individuals with mild influenza disease or severe influenza disease, with neurological or respiratory (RD) complication.
   A. Heatmap displaying the relative abundance of the 308 detected OTUs (rows) for each sample (in column). Relative abundance is shown with a black to white gradient scale with OTUs that were not quantified or with a relative abundance of 10⁻¹⁰ are in white, and OTUs with relative abundance of 1 in black.
   B. Barplot showing relative abundance (in %) of the 10 most abundant OTUs in each sample (in column), classified by genus: *Haemophilus, Moraxella*, *Prevotella*, *Pseudomonas*, *Staphylococcus*, *Streptococcus*, and *Trophyrema*, and further colored by species as indicated in the figure. White bars are for OTUs that were classified only up to the genus level. This barplot is illustrative of the numbers presented in figures 2C and 2D.
   C. Table showing the relative abundance (in %) of the 10 most abundant OTUs in each sample of the individuals with mild disease (in column), classified by genus and species; NA means "not available".
   D. Table showing the relative abundance (in %) of the 10 most abundant OTUs in each sample of the individuals with severe disease (in column), classified by genus and species; NA means "not available".

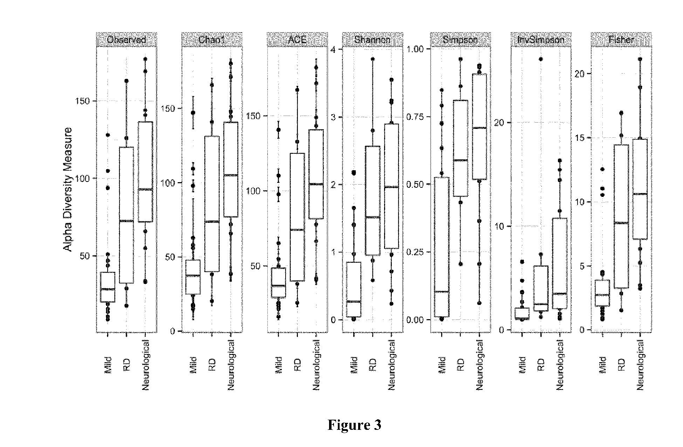

**Figure 3****:** Alpha diversity plots compare different group of patients: Mild, RD (respiratory distress) and Neurological (complication). The following diversity indices were calculated using the phyloseq R package: Observed, Chao1, ACE, Shannon, Simpson, InvSimpson and Fisher indices.

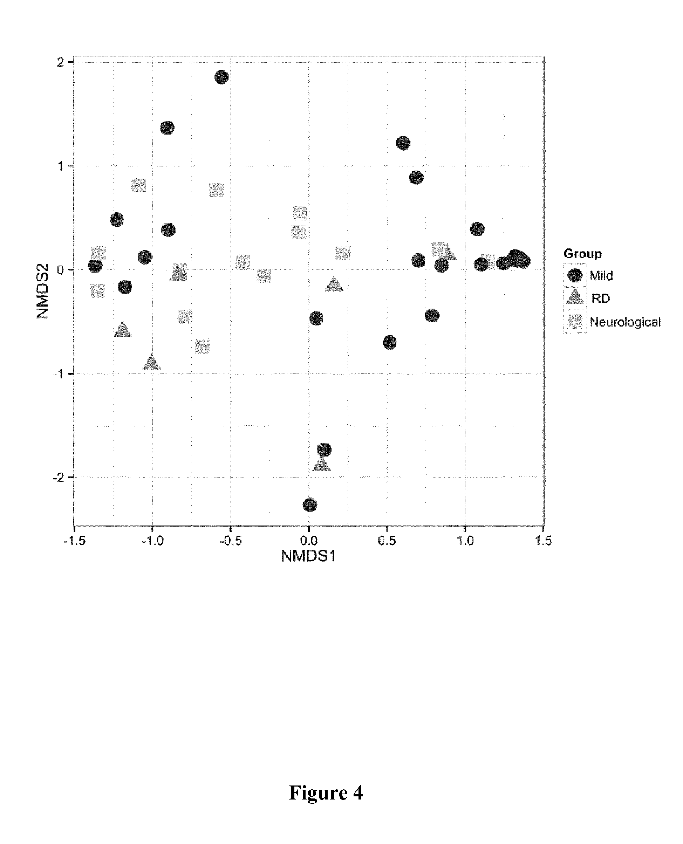

**Figure 4** **:** The respiratory microbiome differentiates influenza severity.
   Non-metric Multidimensional Scaling (NMDS) plot comparing the global respiratory microbial profiles with distances calculated using the Bray-Curtis method. Each dot represents a sample, colored and shaped according to influenza outcome:
   - patients with mild influenza disease are displayed in dark grey circles,
   - patients with respiratory complication (RD) are in medium grey triangles,
   - patients with neurological complication are in light grey squares.
   Patients with similar microbiome profiles are close together in the NMDS plot, while increasing distances in the plot between samples suggest divergent microbiome profiles.

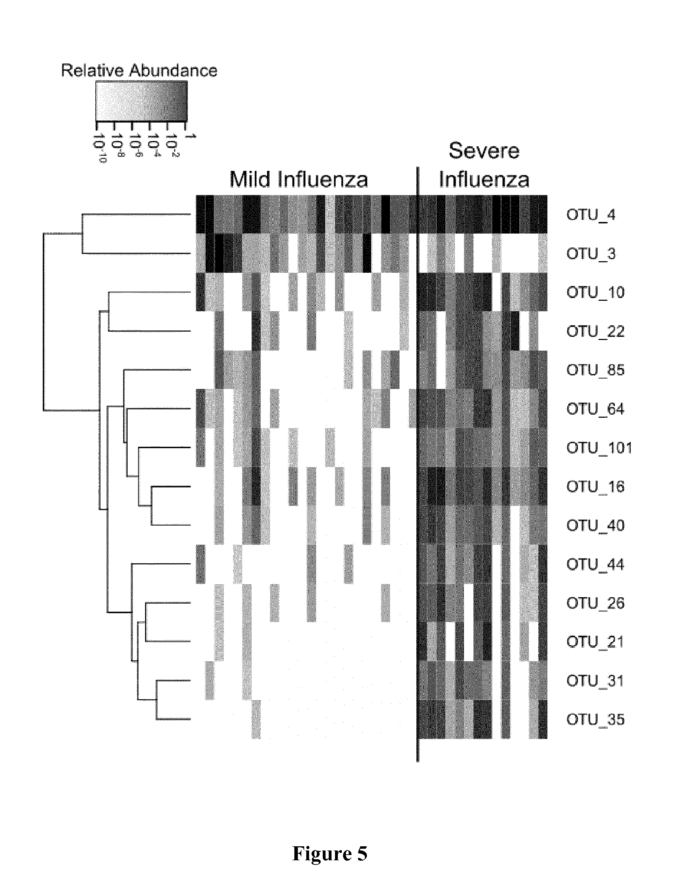

**Figure 5** **:** Bacterial species are differentially abundant between patients with mild and severe influenza.
   Heatmap is showing the relative abundance of 14 OTUs that were found differentially abundant between mild and all severe patients after adjusting for age (p-value < 0.05). Relative abundance is shown with a black to white gradient scale, with OTUs that were not quantified, or with a relative abundance of 10⁻¹⁰ being in white, and OTUs with a relative abundance of 1 being in black.

### DETAILED SPECIFICATION OF THE INVENTION

The present invention relates to the identification of new biomarkers, selected among (i) the alpha diversity value of the microbiome present in a respiratory sample of an individual and (ii) the microbiome profile of said respiratory sample, useful for predicting the severity of the disease caused by the infection of an individual with an influenza virus.

### Infection with an influenza virus

In the sense of the invention "influenza infection" designates the presence of influenza virus in a sample of an individual that is designated hereafter as an "individual infected with an influenza virus", an "influenza-infected individual" or "a patient".

The infection is generally confirmed by testing the throat, sputum, or nose for the presence of at least one influenza virus.

In the sense of the invention, the term "influenza virus" designates a virus chosen among influenza virus type A, B and C, and mutants thereof. Influenza viruses contemplated herein include viruses that have two antigenic glycosylated enzymes on their surface: neuraminidase and hemagglutinin. Various subtypes of influenza virus of the invention include, but are not limited to, the H1N1, H1N2, H2N2, H3N2, H3N8, H5N1, H5N3, H5N8, H5N9, H7N1, H7N2, H7N3, H7N4, H7N7, H7N9, H9N2, and H10N7 subtypes including the following subtypes commonly known as the "Spanish Flu," "Asian Flu," "Hong Kong Flu," "Avian Flu," "Swine Flu," "Horse Flu," and "Dog Flu."

An influenza-caused disease lasts generally seven to ten days. Symptoms of the influenza-caused disease are generally apparent one to four days after the virus enters the body, i.e. the infection. In certain cases, a person can be infected with the flu virus but demonstrate no symptoms. During this time, those persons can still transmit the virus to others.

An "infection with an influenza virus" must be distinguished from the "disease caused by an influenza infection", also denominated in the present application "influenza disease", "influenza related disease", "influenza-caused disease", "influenza" or "disease", that means the pathology, i.e. the group of symptoms that usually affect individuals being infected with the influenza virus.

The most common symptoms of the influenza related disease include: a high fever, runny nose, sore throat, muscle pains, headache, coughing, and intense tiredness.

In particular, it is to be understood that the intensity of an "influenza infection", meaning a high viral load in a respiratory sample of the infected-individual, does not necessarily correlate with the severity of the "influenza-caused disease" that manifests as a result of the infection.

### Severity of the disease

During the influenza-caused disease, symptoms can be mild to severe. In particular, the clinical outcome of the infection can necessitate hospitalizations of certain infected-individuals, if complications occur.

In the sense of the present invention, the term complications refers to medical/clinical problems that are observed in individuals diagnosed with an influenza infection. Complications associated with an influenza infection include, without limitation:
- respiratory complications such as bronchitis, tracheitis, rhinitis, sinusitis, asthma, primary viral pneumonia that can lead to acute respiratory distress syndrome (ARDS), secondary bacterial pneumonia, croup, otitis media, pulmonary fibrosis, obliterative bronchiolitis, bronchiectasis, exacerbations of asthma, exacerbations of chronic obstructive pulmonary disease, lung abscess, empyema, pulmonary aspergillosis;
- cardiac complications such as atrial fibrillation, myocarditis, pericarditis, heart failure;
- neurological complications such as confusion, convulsions, psychosis, neuritis, Guillain-Barre syndrome, coma, transverse myelitis, encephalitis, encephalomyelitis, Reye's syndrome;
- toxic shock syndrome; myositis; myoglobinuria; renal failure; and
- early and late fetal deaths in pregnant women, increased perinatal mortality in pregnant women, and congenital abnormalities in birth.

Complications of the influenza-caused disease include in particular respiratory and neurological complications.

Basically, in the vocabulary of the medical personnel, the term of "severe disease" designates the cases wherein a hospitalization of the infected individual is required, for any of the complication listed above.

More particularly, common criteria used for classifying influenza-infected individuals into the group of "individuals with severe influenza disease" also called "individuals developing a severe influenza disease" include:
- when the severity of the disease is related to a respiratory complication: utilization of invasive or non-invasive ventilation, bleed gas alteration (hypoxemia <95% in arterial sample), and hospitalization in ICU for respiratory complication;
- when the severity of the disease is related to a neurological complication: neurological symptoms such as faint, convulsion, or abnormal comportments, and/or results of examination in Magnetic Resonance Imagery (MRI), Electroencephalogram, Cerebrospinal Fluid (LCS) perturbations.

In the sense of the invention, the term "predicting the severity of a disease caused by the infection of an individual with an influenza virus" designates the evaluation of the risk that influenza-infected individuals might present, in the clinical evolution of the disease, a complication, in particular at least one respiratory and/or neurological complication.

An influenza-caused disease lasts generally seven to ten days. Usually, the first symptoms appear one or two days after the actual infection, corresponding to the integration of the virus into the cells of an individual.

A severe clinical evolution of the influenza-caused disease might happen after one, two, three, four, five, six, seven, eight, nine, ten, eleven, twelve, thirteen or fourteen days after the infection occurs.

A severe clinical evolution of the influenza-caused disease might also happen after one, two, three, four, five, six, seven, eight, nine, ten, eleven, twelve, thirteen or fourteen days after the individual has been presenting the first influenza disease symptoms.

In a specific embodiment of the use according to the invention, the severe disease is characterized by the occurrence of at least one respiratory complication.

The term "respiratory complication" relates to the occurrence of at least one episode of bronchitis, tracheitis, rhinitis, sinusitis, asthma, primary viral pneumonia that can lead to acute respiratory distress syndrome (ARDS), secondary bacterial pneumonia, croup, otitis media, pulmonary fibrosis, obliterative bronchiolitis, bronchiectasis, exacerbations of asthma, exacerbations of chronic obstructive pulmonary disease, lung abscess, empyema, pulmonary aspergillosis.

In a specific embodiment of the methods according to the invention, the severe disease is characterized by the occurrence of at least one episode of respiratory distress (RD). The term "respiratory distress" also designated as "acute respiratory distress" (RD or ARD) is defined as modification of breathing rate, increased heart rate, color changes, grunting, nose flaring, retractions, sweating wheezing, stridor, accessory muscle use, or changes in alertness.

The term "acute respiratory distress syndrome" (ARDS) refers to a severe, life-threatening medical condition characterized by a widespread inflammation in the lungs, that leads to a decreased gas exchange. This syndrome is associated with a high mortality rate, between 20 and 50% of the affected individuals.

In a specific embodiment of the use according to the invention, the severe disease is characterized by the occurrence of at least one neurological complication.

The term "neurological complication" relates to the occurrence of at least one episode of confusion, convulsions, psychosis, neuritis, Guillain-Barre syndrome, coma, transverse myelitis, encephalitis, Reye's syndrome or encephalomyelitis in an individual infected with an influenza virus.

### Biomarker

A biomarker generally refers to a measurable indicator of some biological condition, or an indicator of a future biological condition.

In the sense of the invention, the term "biomarker" designates a marker measurable in a biological sample of an individual infected with an influenza virus, being an indicator of the clinical outcome of the influenza-caused disease in said individual.

The "clinical outcome" designates the level of severity of the influenza disease in the days after the measure of the biomarker, meaning after at least one, two, three, four, five, six, seven, eight, nine, ten, eleven, twelve, thirteen or fourteen days after the day the biomarker is measured.

In particular the "clinical outcome" includes the occurrence, or not, of a respiratory or neurological complication.

The present invention relates to the use of at least one biomarker chosen among (i) the alpha diversity value of the microbiome present in a respiratory sample of an individual and (ii) the microbiome profile of the said respiratory sample, for predicting the severity of an influenza-caused disease in a tested individual, in the days after the measure of the biomarker.

In an embodiment of the invention, the invention relates to the use of the alpha diversity value of a microbiome present in a respiratory sample of an individual infected with an influenza virus, as a biomarker for predicting the severity of the influenza-caused disease in said individual, in the days after the measure of the biomarker.

In another embodiment of the invention, the invention relates to the use of the microbiome profile, corresponding to the taxonomic profile and the relative microbial abundance, determined in a respiratory sample of an individual infected with an influenza virus, as a biomarker for predicting the severity of the influenza-caused disease in said individual in the days after the measure of the biomarker.

In a particular embodiment of the invention, the invention relates to the use of both biomarkers, the alpha diversity value and the microbiome profile present in a respiratory sample of an individual infected with an influenza virus, for predicting the severity of the influenza-caused disease in said individual in the days after the measure of the biomarker.

In the sense of the invention, the terms "microbiome", "microbial community", "bacterial community" and "microbiota" designate the community of microorganisms, including symbiotic, pathogenic and commensal (i.e. normally present and harmless in small quantities) microorganisms, that are present in the human body.

Microbiomes are present in every part of the human body; each microbiome presents a microbiome profile that is function of the organ, mucosal surface or biological fluid where it is found. Microbiome profiles are also function, among other factors, of the food intake and of the hygiene habits of the individual.

In the sense of the invention, "microbiome profile" designates the composition of the microbial community in a site, both qualitatively and quantitatively. This term regroups the notions of taxonomic classification, i.e. the genus and species present in said microbiome, and the relative abundance of each identified genus and species.

In the sense of the invention, "respiratory microbiome profile" designates the composition of the microbial community in a respiratory site, both qualitatively and quantitatively.

Specific microbiome profiles have been linked to human pathological conditions. For example, the patent application WO 2008/076696 relates to the gut microbiome profile as a biomarker and therapeutic target for promoting weight loss in a subject. In the same idea, the patent application WO 2013/176774 discloses a method of treating an individual having autism spectrum disorder, comprising reintroducing into the gut microbiome of said individual specific bacterium being present in a relatively low abundance, compared to a reference gut microbiome.

The normal upper respiratory tract microbiota has not received the same intense study as other body sites; nevertheless, a basic picture of this community has emerged. The current consensus is that a healthy noise and nasopharynx presents a bacterial community dominated by Actinobacteria, Firmicutes, and Proteobacteria. At the genus level, *Corynebacterium* spp., *Propionibacterium* spp. and *Staphylococcus* spp. are prominent members of this microbiome.

An imbalance of the oropharyngeal microbiota in H1N1 influenza-infected patients, compared to healthy individuals, has been reported: *Pseudomonas, Bacillus* and *Ralstonia* species, equipped with chemotaxis and flagellar assembly genes, increases significantly in the microbial community of H1N1-infected group (Leung *et al.,* 2012). Authors conclude that the influenza infection, by weakening immunity, allows the access to the respiratory tract for microbial pathogens.

Characterization of the upper respiratory tract microbiomes of H1N1 influenza-infected individuals has been reported by Chaban and collaborators (Chaban *et al.,* 2013). The conclusion of this study is that the observed microbiome profile could not be correlated with any of the patient characteristics, except for an increased diversity of the microbiome with patient age.

### Biological sample

The biomarkers of the present invention are measured in a respiratory sample of an influenza-infected individual.

In the sense of the invention, the term "respiratory sample" means a biological sample originating from the respiratory tract of an individual. This sample originates from the upper respiratory tract, comprising the nasal cavity, paranasal sinuses, the pharynx and the larynx, or from the lower respiratory tract, comprising the trachea, the primary bronchi and the lungs.

According to a specific embodiment of the invention, the respiratory sample is selected from a nasopharyngeal aspirate, a nasal swab, a throat swab, a broncho-alveolar lavage and a tracheobronchial aspirate.

Advantageously, the use of the biomarker of the invention allows predicting the severity of the influenza-caused disease at an early stage of the disease, and preferentially at the beginning of the influenza disease symptoms, as listed above.

It is understood that the prediction of the severity of the disease occurs before the clinical symptoms of a severe disease occur, for example one day, two days, three days, four days, five days, six day, seven days, eight days, nine days or ten days before the hospitalization of the infected individual, and/or before the occurrence of an episode of a respiratory and/or neurological complication.

Therefore, it is advantageous, for the implementation of the invention, that the respiratory sample be obtained and analyzed at an early stage of the influenza-caused disease.

According to a preferred embodiment of the invention, the respiratory sample originates from an individual that has been presenting influenza disease symptoms for about 24 to 72 hours, preferably from less than 48 hours.

Preferably, the respiratory sample is obtained by a qualified person in the adequate conditions of hygiene, and is conserved in appropriate conditions for subsequent studies of the alpha diversity and/or microbiome profile present in said respiratory sample.

### Alpha diversity

The present invention also relates to a method for predicting the severity of a disease caused by the infection of an individual with an influenza virus, comprising the steps of:
a) determining the alpha diversity value of the microbiome present in a respiratory sample from the tested individual, and
b) comparing the alpha diversity value determined at step a) to a reference alpha diversity value.

In the sense of the present invention, alpha diversity is a measure of number of species (richness) and their proportional abundance (eveness) in a biological sample.

Different indices representing the alpha diversity value of a microbiome are known by the man skilled in the art, and can be used for the implementation of the uses and methods of the invention, as shown in figure 3.

Accordingly, it is understood that the term "determining the alpha diversity value of the microbiome present in a respiratory sample" means that at least one value of alpha diversity is calculated, and compared to the same value calculated for the reference microbiome profile.

Preferentially, at least one alpha diversity value is determined by using the phyloseq R package software (McMurdie and Holmes, 2013).

Several diversity values can be calculated using this phyloseq R package, that uses the following definitions:
- Chao: S_P = S_0 + a1^2/(2*a2) * (N-1)/N
   with S_P is the extrapolated richness in a pool, S_0 is the observed number of species in the collection, a1 and a2 are the number of species occurring only in one or only in two sites in the collection, , and N is the number of sites in the collection.
- ACE: S_P = S_abund + S_rare/C_ace + al/C_ace * gamma^2 where C_{ace} = 1- a1/N_{rare} and gamma^2 = max(S_rare/C_ace (sum[i=1..10] i*(i-1)*a_i) / N_rare/(N_rare-1) -1 , 0)
   with a_i refers to number of species with abundance I, and S_rare is the number of rare species, S_abund is the number of abundant species, with an arbitrary threshold of abundance 10 for rare species, and N_rare is the number of individuals in rare species.
- Shannon: Shannon or Shannon-Weaver (or Shannon-Wiener) index is defined as H = -sum p_i 1n(p_i),
   where p_i is the proportional abundance of species i.
- Simpson: Simpson's index = 1-D with D = sum p_i^2.
- InvSimpson is 1/D with D = sum p_i^2.
- Fisher is the α parameter of Fisher's logarithmic series and is used as a diversity index.

According to a specific embodiment of the invention, the "alpha diversity value of the microbiome" is the Shannon index (H).

The method according to the invention comprises a step (b) of comparison of the alpha diversity value of a microbiome of a tested individual, with a reference alpha diversity value. Naturally, said reference value will be calculated on a reference respiratory microbiome profile according to the same method of calculation and/or based on the same diversity index, than the one used for the tested microbiome profile.

The "tested individual" refers to an individual infected with at least one influenza virus, from which originates a biological sample that is tested for determining the alpha diversity value of the present microbiome, and whose severity of the disease is not known yet.

According to an embodiment of the invention, the reference alpha diversity value is determined on at least one microbiome present in a respiratory sample of an individual presenting a mild disease caused by an infection with an influenza virus.

According to another embodiment of the invention, the reference alpha diversity value is the mean of at least two alpha diversity values determined on at least two microbiomes present in respiratory samples of at least two individuals presenting a mild disease caused by an infection with an influenza virus.

In a preferred embodiment of the invention, the reference alpha diversity value is the mean of at least three alpha diversity values determined on at least three microbiomes present in respiratory samples of at least three individuals presenting a mild disease caused by an infection with an influenza virus.

The inventors have observed that a higher alpha diversity value of the microbiome present in the tested individual, comparatively with the reference value, is indicative of a risk, for said individual, of developing a severe disease in the next days.

The term "higher" indicates that the alpha diversity value of the microbiome present in the tested individual is strictly superior to the reference value as defined above.

In an embodiment of the invention, a "higher" value can be at least equal to 1,5 times the value of reference, at least about 1,75 times, at least about 2 times, or at least about 2,5 times the value of reference.

In an embodiment of the invention, the method for predicting the severity of an influenza disease in a tested individual comprises the determination of a reference alpha diversity value on at least one microbiome present in a respiratory sample of an individual presenting a mild disease caused by an infection with an influenza virus, wherein a higher alpha diversity value of the microbiome present in said tested individual is indicative of a risk, for said individual, of developing a severe disease.

In an embodiment of the invention, the alpha diversity value is determined on respiratory sample(s) originating from individual(s) infected with an influenza virus, having developed a mild disease, wherein the respiratory sample(s) have been obtained when the individual(s) presented influenza disease symptoms for about 24 to 72 hours, preferably for less than 48 hours.

In a specific embodiment of the invention, the respiratory sample originates from an individual aged of less than 15 years, infected with an influenza virus that has been presenting influenza infection symptoms for about 24 to 72 hours, preferably from less than 48 hours.

### Microbiome profile

The present invention also relates to a method for predicting the severity of a disease caused by the infection of an individual with an influenza virus, comprising the steps of:
a) determining the microbiome profile present in a respiratory sample from the tested individual, and
b) comparing said microbiome profile determined in step a) to a reference respiratory microbiome profile.

Different techniques are known by the man skilled in the art for determining this microbiome profile, representing both the "qualitative" taxonomic profile, and the relative abundance of each species which might be designated as the "quantitative" profile.

In particular, the microbiome can be determined by any method known by the man skilled in the art, such as sequencing the bacterial DNA encoding for 16S RNA hypervariable regions (V1 to V9) contained in a sample, or by whole genome shotgun sequencing of said DNA.

When sequencing one or more hypervariable regions of 16S DNA, the qualitative taxonomic profile is determined using the following steps:
- reads quality filtering and demultiplexing,
- paired reads merging,
- OTU (Operational taxonomic unit) clustering, typically at a 97% identity threshold,
- chimera filtering, and
- taxonomy assignment, by comparing OTU representative sequences to databases such as SILVA database or greengenes.

These different steps can be performed using softwares such as QIIME, mothur or UPARSE.

The "quantitative" microbiome profile is determined by the measure of the relative abundance of each OTU identified. An OTU table that gives the number of reads from each sample that is assigned to each OTU is created by mapping the reads to OTUs, The relative abundance for OTU (i) in sample (s) is then defined as the ratio of number of reads mapping to OTU (i) to the total number of reads of sample (s).

Shotgun metagenomic sequencing is an alternative approach to 16S sequencing, where all DNA present in the sample is fragmented and independently sequenced. The analysis steps performed to assess microbiome profiles from such data include 3 different methods used after reads quality control procedures:
(i) marker gene analyses (involving comparing each read to a reference database of taxonomically or phylogenetically informative sequences);
(ii) a binning metagenomes method (including compositional binning, similarity binning, and fragment recruitment), and
(iii) de novo assembly (reads are merged into contigs and blasted against reference databases to identify species).

Specific microbiome profiles can also be assessed using quantitative PCR targeting specific bacterial species or genus.

The method according to the invention comprises a step (b) of comparison of the respiratory microbiome profile of a tested individual with a reference respiratory microbiome profile.

The "tested individual" refers to an individual infected with at least one influenza virus, from which originates a biological sample that is tested for determining the profile of the present microbiome, and whose severity of the disease is not known yet.

A "reference respiratory microbiome profile" is a set of values corresponding to the relative abundance of about 10 to 20 operational taxonomic units (OTUs) observed in at least one microbiome present in a respiratory sample of an individual presenting a mild disease caused by an infection with an influenza virus. The relative abundance of a given OTU is expressed in percentage or in ratio of the number of reads mapping the OTU over the total number of reads mapping all OTU in a given sample. The reference respiratory microbiome profile is expressed as a set of relative abundance values for the 10 to 20 most abundant OTUs present in said microbiome.

According to an embodiment of the invention, the reference respiratory microbiome profile is a set of relative abundance values, determined on at least one microbiome present in a respiratory sample of an individual presenting a mild disease caused by an infection with an influenza virus.

According to another embodiment of the invention, the reference respiratory microbiome profile is a set of mean values determined on at least two respiratory microbiome profiles present in respiratory samples of at least two individuals presenting a mild disease caused by an infection with an influenza virus.

In a preferred embodiment of the invention, the reference respiratory microbiome profile is a set of mean values determined from at least three respiratory microbiome profiles determined on at least three respiratory samples of at least three individuals presenting a mild disease caused by an infection with an influenza virus.

By "set of mean values", it is referred to a group of values representing the relative abundance of about 10 to 20 OTUs, with each value being the mean of OTU relative abundance values observed in at least two microbiome profiles. With this determination of "mean relative abundance values", a reference respiratory microbiome profile can be defined as a set of mean relative abundance of about 10 to 20 OTUs

In an embodiment of the invention, the reference respiratory microbiome profile is determined from at least one respiratory sample of an individual presenting a mild disease caused by an infection with an influenza virus.

In another embodiment of the invention, the reference microbiome profile is determined in respiratory samples originating from individuals infected with at least one influenza virus, said individuals having developed in mild disease, wherein the respiratory samples have been obtained when the individuals presented influenza disease symptoms for about 24 to 72 hours, preferably for less than 48 hours.

In a specific embodiment of the invention, the respiratory sample of the tested individual, and the respiratory sample(s) used for determining the reference respiratory microbiome profile, originate from individuals aged of less than 15 years, infected with an influenza virus, that have been presenting influenza infection symptoms for about 24 to 72 hours, preferably from less than 48 hours.

### Divergence of the respiratory microbiome profiles.

The inventors have observed that a divergent respiratory microbiome profile present in the tested individual, comparatively with a reference respiratory microbiome profile, is indicative of a risk, for said individual, of developing a severe disease in the next days.

The term "divergent" indicates that the respiratory microbiome profile present in the tested individual is divergent, in terms of relative abundance of at least one OTU, to the reference respiratory microbiome profile as defined above.

The term "divergence" indicates the different relative abundance for at least one OTU comprised in the respiratory microbiome profile.

In an embodiment of the invention, at least one divergence between the respiratory microbiome profile present in the tested individual and the reference respiratory microbiome profile is indicative of a clinical risk, for said individual, to develop a severe disease.

In another embodiment of the invention, at least two, three, four or five divergences between the respiratory microbiome profile present in the tested individual and the reference respiratory microbiome profile are indicative of a clinical risk, for said individual, to develop a severe disease.

In particular, divergences have been observed between the respiratory microbiome profiles present in influenza infected-individuals having developed a severe disease, comparatively with the reference respiratory microbiome profile determined on infected-individuals having developed a mild disease, such as presented in the tables 1 and 2 in the examples.

Tables 1 and 2 show in particular that:
- a lower relative abundance of the bacterium of the *Staphylococcus aureus* species has been observed in the respiratory microbiome profile of individuals having developed a severe disease;
- a higher relative abundance of the bacteria presented in table 1 has been observed in the respiratory microbiome profile of individuals having developed a severe disease; in particular a higher relative abundance value for the following OTUs has been noted: *Prevotella melaninogenica; Neisseria; Prevotella; Streptobacillus; Campylobacter concisus; Porphyromonas ; Capnocytophaga ; Prevotella pallens ; Abiotrophia para-adiacens ; Veillonella dispar ; Solobacterium; Haemophilus sp.; Catonella sp.; Granulicatella elegans ;* and *Streptococcus infantis.*
- a higher relative abundance of the bacteria presented in table 2 has been observed in the respiratory microbiome profile of individuals having developed a severe disease, wherein the statistical test shown in Table 1 has been adjusted for the age of the tested individuals using a generalized linear model; in particular a higher relative abundance value for the following OTUs has been noted: *Streptococcus ; Prevotella melaninogenica ; Prevotella ; Prevotella sp. ; Streptobacillus; Porphyromonas ; Prevotella pallens ; Abiotrophia para-adiacens; Veillonella dispar; Haemophilus sp.; Granulicatella elegans*

As a result, it appears that the following OTUs are, both from results of table 1 and table 2, in a higher relative abundance in the microbiomes of influenza-infected individuals having developed a severe disease: *Prevotella melaninogenica; Prevotella; Porphyromonas ; Prevotella pallens ; Abiotrophia para-adiacens ; Veillonella dispar; Haemophilus sp.; and Granulicatella elegans.*

In an embodiment, the invention relates to a method for predicting the severity of a disease caused by an influenza virus, wherein a lower relative abundance of the bacterium of the *Staphylococcus aureus* species in the microbiome profile in a respiratory sample of a tested individual, compared to a reference respiratory microbiome profile, is indicative of a risk for said tested individual of developing a severe disease

In another embodiment, the invention relates to a method for predicting the severity of a disease caused by an influenza virus, wherein a higher relative abundance of at least one type of bacterium listed in anyone of table 1 and table 2 in the microbiome profile of a respiratory sample of said tested individual, compared to a reference respiratory microbiome profile, is indicative of a risk for said tested individual of developing a severe disease.

In another embodiment, the invention relates to a method for predicting the severity of a disease caused by an influenza virus, wherein a higher relative abundance of at least one type of bacterium selected in a group comprising (i) the genus *Streptococcus, Prevotella, Streptobacillus, Porphyromonas, Haemophilus* and (ii) the species *Abiotnophia para-adiacens, Veillonella dispar,* and *Granulicatella elegans,* in the microbiome profile of a respiratory sample of said tested individual, compared to a reference respiratory microbiome profile, is indicative of a risk for said tested individual of developing a severe disease.

In a specific embodiment of the invention, the tested respiratory sample originates from an individual infected with an influenza virus that has been presenting influenza infection symptoms for about 24 to 72 hours, preferably from less than 48 hours.

In a specific embodiment of the invention, the respiratory sample of the tested individual, and the respiratory sample(s) used for determining the reference respiratory microbiome profile, originate from individuals aged of less than 15 years, infected with an influenza virus, that have been presenting influenza infection symptoms for about 24 to 72 hours, preferably from less than 48 hours.

### Therapeutic uses

The present invention also relates to the use of a bacterium or an extract thereof, as a probiotic, in the treatment and/or prevention of a severe disease in an individual infected with an influenza virus, wherein said bacterium has been identified as being present in a lower relative abundance in the microbiome profile present in a respiratory sample from an individual infected with an influenza virus, and presenting a severe disease, compared to a reference respiratory microbiome profile present in at least one respiratory sample of an individual infected with an influenza virus, and presenting a mild disease.

The use of a bacterium or an extract thereof as a probiotic for therapeutic goals is well known by the man skilled in the art.

In particular, the bacterium or extract thereof can be part of pharmaceutical compositions. Such compositions can be used in a variety of routes of administration, including, for example, orally-administrable forms such as tablets, capsules, inhalation powder or the like, or via parenteral, intravenous, intramuscular, transdermal, buccal, nasal, subcutaneous, suppository, or other route.

In these compositions, the bacterium or extract thereof shall be in an "effective amount" this term referring to the amount necessary to elicit the desired biological response, i.e. the amount necessary to prevent/delay/ameliorate the onset of complications in patients with increased risk for presenting complications associated with an influenza infection.

The present invention also relates to a method for treating and/or preventing a severe disease in an individual infected with an influenza virus, comprising the administration of a bacterium or an extract thereof, wherein said bacterium has been identified as being present in a lower relative abundance in the respiratory microbiome profile present in a respiratory sample from said individual infected with an influenza virus, and presenting a severe disease, compared to a reference respiratory microbiome profile present in at least one respiratory sample of an individual infected with an influenza virus, and presenting a mild disease.

The present invention also relates to the use of an antibiotic specific of a bacterium, in the treatment and/or prevention of a severe disease in an individual infected with an influenza virus, wherein said bacterium has been identified as being present in a higher relative abundance in the respiratory microbiome profile present in a respiratory sample from an individual infected with an influenza virus, and presenting a severe disease, compared to a reference respiratory microbiome profile present in at least one respiratory sample of an individual infected with an influenza virus, and presenting a mild disease.

The use and choice of an antibiotic specifically directed against a bacterium is well known by the man skilled in the art.

In particular, said antibiotic can be part of pharmaceutical compositions. Such compositions can be used in a variety of routes of administration, including, for example, orally-administrable forms such as tablets, capsules, inhalation powder or the like, or via parenteral, intravenous, intramuscular, transdermal, nasal, buccal, subcutaneous, suppository, or other route.

In these compositions, the antibiotic shall be in an "effective amount" this term referring to the amount necessary to elicit the desired biological response, i.e. the amount necessary to prevent/delay/ameliorate the onset of complications in patients with increased risk for presenting complications associated with an influenza infection.

The present invention also relates to a method for treating and/or preventing a severe disease in an individual infected with an influenza virus, comprising the administration of an antibiotic specifically directed against a bacterium, wherein said bacterium has been identified as being present in a higher relative abundance in the respiratory microbiome profile present in a respiratory sample from said individual infected with an influenza virus, and presenting a severe disease, compared to a reference respiratory microbiome profile present in at least one respiratory sample of an individual infected with an influenza virus, and presenting a mild disease.

### EXAMPLES

The following examples illustrate procedures for practicing the invention. These examples should not be understood as limiting the scope of the invention.

The study presented herein is based on respiratory samples obtained from a retrospective clinical collection of children infected with influenza virus, used to compare respiratory microbiome communities between patients with different disease outcomes, mild or severe (respiratory or neurological complications).

### Material and Methods

### Ethics statement

Respiratory samples were collected for regular disease management during hospital stay and no additional samples were taken. For the purpose of this study, patient confidentiality was strictly protected. This study was approved by the ethical committee of Hospices Civils de Lyon.

### Patient selections

We have analyzed 322 clinical records from children (<15 years) admitted in a pediatric hospital (Hôpital Femme-Mère-Enfant, Hospices Civils de Lyon, France) in 2010-2014 and tested positive for influenza A virus. Patients' selection criteria were: age ≤15 years, respiratory sample collected within 2 days of symptoms onset, and hospitalization in intensive care unit (ICU) for acute respiratory distress (ARD) or neurological complications (group of severe influenza) or discharged patients with mild disease (age- and sex-matched patients with mild influenza).

Criteria used for classifying children in the group of severe influenza with respiratory complication were: utilization of invasive or non-invasive ventilation, bleed gas alteration (hypoxemia <95% in arterial sample), and hospitalization in ICU for respiratory complication. Children were classified in the group of severe influenza with neurological manifestation if they presented: neurological symptoms (faint, convulsion, or abnormal comportments), Magnetic Resonance Imagery (MRI), Electroencephalogram, Cerebrospinal Fluid (LCS) perturbations (Wilking *et al.,* 2014). All ambiguous cases were classified using PRISM III score described by Pollack *et al* (1996). Patients with a PRISM III score higher than 3 were considered as severe cases. We have excluded patients receiving antibiotics prior to respiratory sample collection as this treatment likely perturbs the microbiome and patients with incomplete clinical files.

In total, 72 patients were selected, including 42 patients with mild influenza, 11 patients with respiratory distress symptom and 19 patients with neurological complications. We have extracted DNA and RNA using Nuclisens EasyMag.

Only 37 patients had samples with sufficient DNA quantity and quality for sequencing, including 22 patients with mild influenza, 7 patients with acute respiratory distress symptom and 10 patients with neurological complications.

### 16S rRNA sequencing and analysis

Genomic DNA was extracted from de-identified patient respiratory samples (n=44). Custom metagenomic libraries that amplify the variable regions, V1-V3, of the 16S ribosomal gene were generated and sequenced on an Illumina® MiSeq platform. Each sample had an average of 150K read depth and all 16S ribosomal sequences were classified using the UPARSE metagenomic pipeline. This pipeline QC'ed the raw sequence data, merged the paired reads, removed sequence artifacts (chimeric sequences, sequence errors), mapped full-length reads to a highly curated 16S ribosomal database, identified sequence reads to genus/species level with a 97% cutoff based on OTU classification, and generated summary tables for downstream statistical analyses.

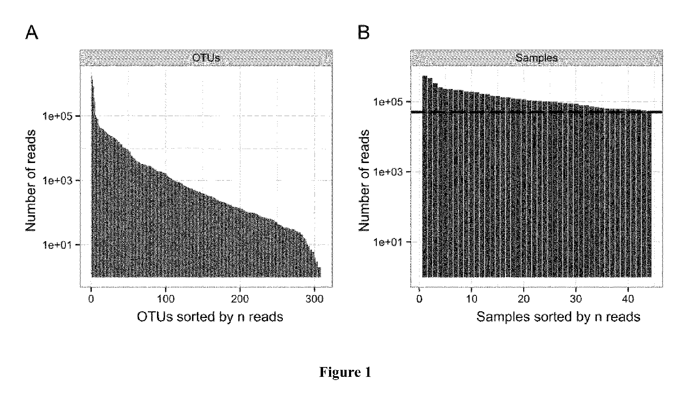

As shown in figure 1, all samples passed our cut-off of at least 50,000 high-quality *16S DNA* sequence reads per sample and 308 OTUs were identified.

### Statistical analysis

All statistical analysis was performed using R Statistical Software. The R "phyloseq" package was used to calculate abundance-based richness estimators (e.g. *Chao1, ACE*), and diversity indices (e.g. *Shannon,* Simpson and Fisher). Association between the Shannon diversity index (H) and patient group was assessed by one way ANOVA with Tukey multiple comparisons of means post hoc test. Multi-factor Analysis of Variance was used to identify covariates associated with H. The R "phyloseq" package was also used to calculate distance metrics (Bray-Curtis and weighted Unifrac) and visualize samples into two dimensions using ordination methods. Beta diversity analysis was performed on relative abundance data (i.e proportions: counts are divided by total library size). Permutational multivariate analysis of variance (PerMANOVA) was performed using the R "vegan" package to test the ability of variables (patient group, gender, age, sample nature, and time since sample onset) to account for observed variance in microbiome profiles.

Finally, statistical analyses for the differential abundance of mapped OTU reads were conducted by the ALDEx2 R package using a Wilcoxon rank test and a FDR (False Discovery Rate) cutoff of 5%. To adjust for patient's age, a linear model was calculated using aldex.glm function of ALDEx2. Differential OTUs were defined using a p-value cutoff of 0.05.

### Diversity measurement

Diversity indices were calculated using the phyloseq R package that used the following definitions:
- Chao: S_P = S_0 + a1^2/(2*a2) * (N-1)/N with S_P is the extrapolated richness in a pool, S_0 is the observed number of species in the collection, a1 and a2 are the number of species occurring only in one or only in two sites in the collection, p_i is the frequency of species i, and N is the number of sites in the collection.
- ACE: S_P = S_abund + S_rare/C_ace + al/C_ace * gamma^2 where C_{ace} = 1- al/N_{rare} and gamma^2 = max(S_rare/C_ace (sum[i=1..10] i*(i-1)*a_i) / N_rare/(N_rare-1) -1,0) with a_i refers to number of species with abundance i and S_rare is the number of rare species, S_abund is the number of abundant species, with an arbitrary threshold of abundance 10 for rare species, and N_rare is the number of individuals in rare species.
- Shannon: Shannon or Shannon-Weaver (or Shannon-Wiener) index is defined as H = -sum p_i ln(p_i), where p_i is the proportional abundance of species i.
- Simpson: Simpson's index = 1-D with D = sum p_i^2.
- InvSimpson is 1/D with D = sum p_i^2.
- Fisher is the α parameter of Fisher's logarithmic series and is used as a diversity index.

### Results

### Respiratory microbiome composition

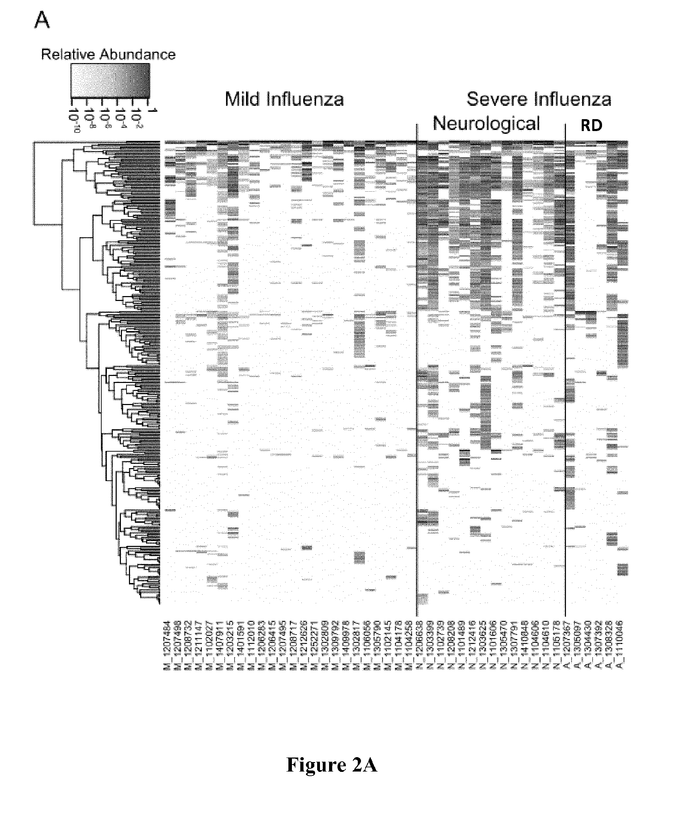

We profiled the respiratory microbiome of 44 respiratory samples, including 37 acute samples collected from 22 patients with mild influenza, 5 patients with respiratory distress symptom and 10 patients with neurological complications. In addition, we used 7 longitudinal samples from 5 different patients. Overall, 308 operational taxonomic units (OTUs) were detected with 49% classified up to the genus level (Fig 2A).

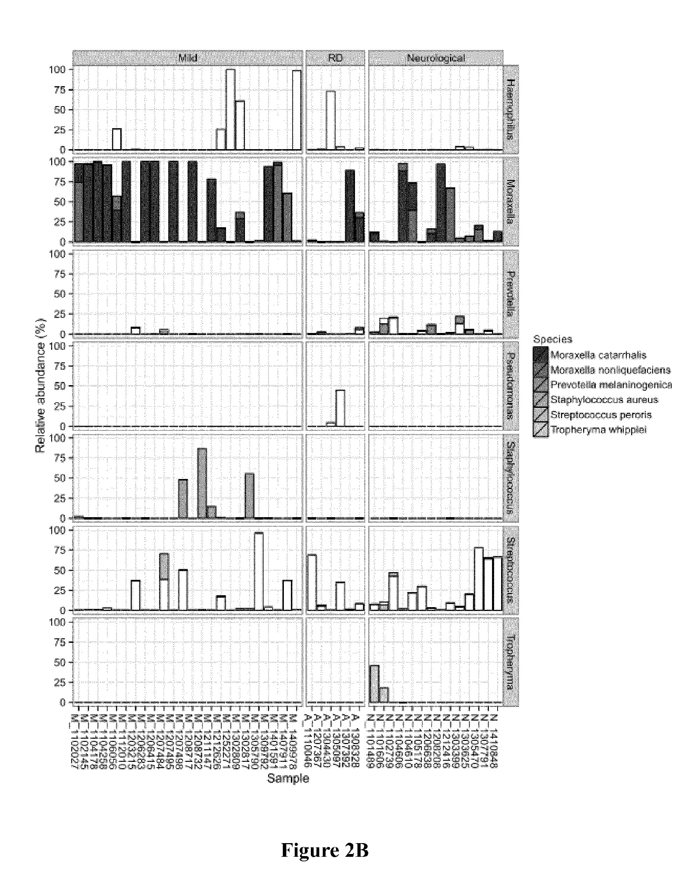

*Moraxella catharrhalis* was found as the most abundant species in children respiratory microbiome, representing between 40 and 70% on average in the neurological group and in the mild influenza group, respectively (Fig 2B, 2C and 2D).

Some bacterial species were found in some children with neurological complication only, as bacteria from the *Prevotella* genus or from the *Trophyrema* genus, or in children with ARDS only, as bacteria from the *Pseudomonas* genus (Fig 2B, 2C and 2D).

### Mild influenza had a less diverse respiratory microbiome than patients that develop severe influenza

Patient samples were group based on influenza disease severity and the respiratory microbiome composition diversity (alpha diversity) was measured with the following indices: Chao1, ACE, Shannon, Simpson, InvSimpson, and Fisher (Fig 3). All diversity indices indicate the same divergences within the samples, and the Shannon diversity index (H) was further used to differentiate species diversity in respiratory samples from each patient group.

Figure 3 shows that severe influenza-associated microbial communities, in general, appear to be more diverse than mild influenza-associated microbial communities.

Respiratory microbiome diversity was highly associated with influenza outcome (one-way ANOVA p-value=0.000163): patients with mild influenza had a significantly lower microbiome diversity (average H=0.62) than patients with ARD (average H=1.86, p-value=0.013), and than patients with neurological complications (average H=1.94, p-value=0.0003). This analysis clearly demonstrates that the microbial species diversity is significantly greater in patients with severe vs. mild influenza infections. Using multiple regression, we identified that microbiome diversity was also associated with patient age, sample nature and time since sample onset (but not with patient gender). After adjusting for these covariates, we found that increased respiratory bacterial diversity was significantly associated with severe influenza.

**The whole respiratory microbiome differentiates influenza severity.**

Different ordination methods and different distances metrics (Bray-Curtis, weighted UniFrac) show that respiratory microbiome profiles cluster samples by influenza clinical outcome, with a few samples being outliers (Fig 4). Analysis of variance using distance matrices revealed that both bray Curtis dissimilarity and weighted Unifrac distance were dependent of influenza outcome (p=0.008 and p=0.004, respectively), patient age (p=0.017 and p=0.003, respectively) and nature of samples (p=0.03 and p=0.07) but not of gender (p=0.31 and p=0.84) and time since symptom onset (p=0.19 and p=0.24325).

### Differential microbial abundance

Statistical analysis to characterize the differential microbial species abundance between patients with mild influenza and patients with severe influenza was performed using a Wilcoxon Rank test.

Eighteen OTUs were found differentially abundant between the two groups of patients (Fig 5A). In the tables, NA means "not available".

Two lists of divergent relative abundance values of OTUs are given in Tables 1 and 2:
- Table 1 shows the relative abundance for 18 OTUs differentiating mild *vs* severe patients using a Wilcoxon rank test and a FDR (False Discovery Rate) cutoff of 5%;
- Table 2 shows the relative abundance for 14 OTUs differentiating mild vs severe patients using a generalized linear model adjusting for patients'age and a p-value cutoff of 0.05.

**Table 1**

| | | | | **Average Relative abundance (%) in patients with:** | | |
|---|---|---|---|---|---|---|
| **OTU** | **Genus** | **Species** | **Strain** | **Mild Influenza** | **Severe Influenza** | **FDR** |
| OTU_3 | *Staphylococcus* | *Staphylococcus aureus* | NA | 8,68% | 0,02% | 0,010 |
| OTU_10 | *Prevotella* | *Prevotella melaninogenica* | ATCC 25845 | 0,27% | 2,44% | 0,013 |
| OTU_11 | *Neisseria* | *NA* | NA | 0,00% | 1,07% | 0,049 |
| OTU_16 | *Prevotella* | *NA* | NA | 0,35% | 2,99% | 0,003 |
| OTU_22 | *Streptobacillus* | *NA* | NA | 0,25% | 1,43% | 0,045 |
| OTU_25 | *Campylobacter* | *Campylobacter concisus* | TNSWCD | 0,01% | 1,00% | 0,029 |
| OTU_26 | *Prevotella* | *NA* | NA | 0,00% | 0,68% | 0,032 |
| OTU_30 | *Porphyromonas* | *NA* | NA | 0,07% | 0,91% | 0,029 |
| OTU_31 | *Porphyromonas* | *NA* | NA | 0,00% | 0,28% | 0,004 |
| OTU_33 | *Capnocytophaga* | *NA* | NA | 0,00% | 0,51% | 0,034 |
| OTU_35 | *Prevotella* | *Prevotella pollens* | NA | 0,00% | 1,16% | 0,032 |
| OTU_40 | *Grunulicatella* | *Abiotrophia para-adiacens* | NA | 0,01% | 0,50% | 0,011 |
| OTU_64 | *Veillonella* | *Veillonella dispar* | ATCC 17748 | 0,05% | 0,74% | 0,007 |
| OTU_71 | *Solobacterium* | *NA* | NA | 0,00% | 0,11% | 0,033 |
| OTU_85 | *Haemophilus* | *Haemophilus sp.* | NA | 0,04% | 0,52% | 0,032 |
| OTU_100 | *Catonella* | *Catonella sp.* | oral clone EZ006 | 0,01% | 0,10% | 0,044 |
| OTU_101 | *Granulicatella* | *Granulicatella elegans* | NA | 0,06% | 0,19% | 0,006 |
| OTU_122 | *Streptococcus* | *Streptococcus infantis* | X | 0,03% | 0,29% | 0,036 |

**Table 2**

| | | | | **Average Relative abundance (%) in patients with:** | | |
|---|---|---|---|---|---|---|
| | **Genus** | **Species** | **Strain** | **Mild Influenza** | **Severe Influenza** | **p-value** |
| OTU_3 | *Staphylococcus* | *Staphylococcus aureus* | NA | 8,68% | 0,02% | 0,005 |
| OTU_4 | *Streptococcus* | *NA* | NA | 12,11% | 23,53% | 0,049 |
| OTU_10 | *Prevotella* | *Prevotella melaninogenica* | ATCC 25845 | 0,27% | 2,44% | 0,013 |
| OTU_16 | *Prevotella* | *NA* | NA | 0,35% | 2,99% | 0,000 |
| OTU_21 | *Prevotella* | *Prevotella sp.* | oral taxon 306 str. F0472 | 0,00% | 0,99% | 0,019 |
| OTU_22 | *Streptobacillus* | *NA* | NA | 0,25% | 1,43% | 0,048 |
| OTU_26 | *Prevotella* | *NA* | NA | 0,00% | 0,68% | 0,027 |
| OTU_31 | *Porphyromonas* | *NA* | NA | 0,00% | 0,28% | 0,006 |
| OTU_35 | *Prevotella* | *Prevotella pallens* | NA | 0,00% | 1,16% | 0,024 |
| OTU_40 | *Grunulicatella* | *Abiotrophia paraadiacens* | NA | 0,01% | 0,50% | 0,016 |
| OTU_44 | *Prevotella* | *Prevotella sp.* | oral clone GI032 | 0,01% | 0,64% | 0,049 |
| OTU_64 | *Veillonella* | *Veillonella dispar* | ATCC 17748 | 0,05% | 0,74% | 0,007 |
| OTU_85 | *Haemophilus* | *Haemophilus sp.* | NA | 0,04% | 0,52% | 0,013 |
| OTU_101 | *Grunulicatella* | *Granulicatella elegans* | NA | 0,06% | 0,19% | c |

Among the divergent OTUs, OTUs classified to the species level included *Staphylococcus aureus* that was more abundant in mild patients than in severe patients.

Other species were found more abundant in severe patients than in mild patients and included *Prevotella malaninogenica* and *Veillonella dispar* which are part of the normal oral and respiratory tract flora and can cause pulmonary infections; and *Granulicatella elegans* and *Abiotrophia para-adiacens* which are *Nutritionally variant Streptococci,* a common component of the oral flora but which have been associated with endocarditis and septicemia.

Among the genus *Prevotella* and *Porphyromonas,* two different OTUs have been identified, even if the species could not be determined.

### REFERENCES

WO 2014/008545
WO 2008/076696
WO 2013/176774
To KKW, Hung IF, Li IW, Lee KL, Koo CK, Yan WW, Liu R, Ho KY, Chu KH, Watt CL, Luk WK, Lai KY, Chow FL, Mok T, Buckley T, Chan JF, Wong SS, Zheng B, Chen H, Lau CC, Tse H, Cheng VC, Chan KH, Yuen KY. Delayed clearance of viral load and marked cytokine activation in severe cases of pandemic H1N1 2009 influenza virus infection. Clin Infect Dis. 2010 Mar 15;50(6):850-9.
Zuniga J, Buendia-Roldan I, Zhao Y, Jimenez L, Torres D, Romo J, Ramirez G, Cruz A, Vargas-Alarcon G, Sheu CC, Chen F, Su L, Tager AM, Pardo A, Selman M, Christiani DC. Genetic variants associated with severe pneumonia in A/H1N1 influenza infection. Eur Respir J. 2012 Mar;39(3):604-10. Epub 2011 Jul 7.
Zhang YH, Zhao Y, Li N, Peng YC, Giannoulatou E, Jin RH, Yan HP, Wu H, Liu JH, Liu N, Wang DY, Shu YL, Ho LP, Kellam P, McMichael A, Dong T. Interferon-induced transmembrane protein-3 genetic variant rs12251-C is associated with severe influenza in Chinese individuals. Nat Commun. 2013;4:1418
McMurdie PJ and Holmes S. "phyloseq: An R package for reproducible interactive analysis and graphics of microbiome census data. " PLoS ONE, (2013) 8(4), pp. e61217
Chaban, B., et al., Characterization of the upper respiratory tract microbiomes of patients with pandemic H1N1 influenza. PLoS One, 2013. 8(7): p. e69559.
Leung, R.K., et al., Modulation of potential respiratory pathogens by pH1N1 vital infection. Clin Microbiol Infect, 2013. 19(10): p. 930-5.
Wilking AN, Elliott E, Garcia MN, Murray KO, Munoz FM. Central nervous system manifestations in pediatric patients with influenza A H1N1 infection during the 2009 pandemic. Pediatr Neurol. 2014 Sep;51(3):370-6.
Pollack, Patel and Ruttimann. PRISM III an updated pediatrick risk of mortality scope. Cit.Care.Med 24 (5) 1996

## Claims

1. The use of at least one biomarker for predicting the severity of a disease caused by the infection of an individual with an influenza virus, wherein said biomarker is selected in a group comprising (i) the alpha diversity value of the microbiome present in a respiratory sample of said individual and (ii) the microbiome profile of the said respiratory sample.

2. Use of a biomarker according to claim 1, wherein the respiratory sample is selected from a nasopharyngeal aspirate, a nasal swab, a throat swab, a broncho-alveolar lavage and a tracheobronchial aspirate.

3. Use of a biomarker according to anyone of claims 1 to 2, wherein the respiratory sample originates from an individual that has been presenting influenza disease symptoms for about 24 to 72 hours, preferably for less than 48 hours.

4. A method for predicting the severity of a disease caused by the infection of an individual with an influenza virus, comprising the steps of :
a) determining the alpha diversity value of the microbiome present in a respiratory sample from the tested individual, and
b) comparing the alpha diversity value determined at step a) to a reference alpha diversity value.

5. The method according to claim 4, wherein the reference alpha diversity value is determined on at least one microbiome present in a respiratory sample of an individual presenting a mild disease caused by an infection with an influenza virus,
and wherein a higher alpha diversity value of the microbiome present in said tested individual is indicative of a risk, for said individual, of developing a severe disease.

6. The method according to claim 4 or 5, wherein the individual infected with an influenza virus has been presenting influenza disease symptoms for about 24 to 72 hours, preferably for less than 48 hours.

7. A method for predicting the severity of a disease caused by the infection of an individual with an influenza virus, comprising the steps of:
a) determining the microbiome profile in a respiratory sample from the tested individual, and
b) comparing said microbiome profile determined in step a) to a reference respiratory microbiome profile.

8. The method according to claim 7, wherein the reference respiratory microbiome profile is determined in at least one respiratory sample of an individual presenting a mild disease caused by an infection with an influenza virus.

9. The method according to claim 8, wherein a lower relative abundance of the bacterium of the *Staphylococcus aureus* species in the respiratory microbiome profile of said tested individual, compared to a reference respiratory microbiome profile, is indicative of a risk for said tested individual of developing a severe disease.

10. The method according to claim 8, wherein a higher relative abundance of at least one type of bacterium selected in a group comprising (i) the genus *Streptococcus*, *Prevotella, Streptobacillus*, *Porphyromonas, Haemophilus* and (ii) the species *Abiotrophia para-adiacens, Veillonella dispar* and *Granulicatella elegans,* in the respiratory microbiome profile of said tested individual, compared to a reference respiratory microbiome profile, is indicative of a risk for said tested individual of developing a severe disease.

11. The method according to anyone of claims 7 to 10, wherein the individual infected with an influenza virus has been presenting influenza disease symptoms for about 24 to 72 hours, preferably for less than 48 hours.

12. The use according to anyone of claims 1 to 3, or the method according to anyone of claims 4 to 11, wherein the severe disease is **characterized by** the occurrence of at least one respiratory complication, in particular of a respiratory distress.

13. The use according to anyone of claims 1 to 3, or the method according to anyone of claims 4 to 11, wherein the severe disease is **characterized by** the occurrence of at least one neurological complication.

14. Use of a bacterium or an extract thereof, in the treatment and/or prevention of a severe disease in an individual infected with an influenza virus, wherein said bacterium has been identified as being present in a lower relative abundance in the respiratory microbiome profile present in at least one respiratory sample from an individual infected with an influenza virus, and presenting a severe disease,
compared to a reference respiratory microbiome profile present in at least one respiratory sample of an individual infected with an influenza virus, and presenting a mild disease.

15. Use of an antibiotic specific of a bacterium, in the treatment and/or prevention of a severe disease in an individual infected with an influenza virus,
wherein said bacterium has been identified as being present in a higher relative abundance in the respiratory microbiome profile present in a respiratory sample from an individual infected with an influenza virus, and presenting a severe disease,
compared to a reference respiratory microbiome profile present in at least one respiratory sample of an individual infected with an influenza virus, and presenting a mild disease.
